# EUROPÄISCHE PATENTSCHRIFT

(11) **EP 1 243 234 B1**
(45) Veröffentlichungstag und Bekanntmachung des Hinweises auf die Patenterteilung: **02.02.2005**
(21) Anmeldenummer: 02003712.3
(22) Anmeldetag: 19.02.2002
(51) Int. Cl.: A61F 2/34, A61B 17/68

(54) **Set für das Erstellen eines Implantats als Ersatz für eine zerstörte Beckenknochenpfanne**
Set for constructing an implant for replacing a damaged acetabular cavity
Ensemble pour réaliser un implant de remplacement d'une cavité acétabulaire endommagée

(30) Priorität: 20.03.2001 DE 10113614
(43) Veröffentlichungstag der Anmeldung: 25.09.2002
(73) Patentinhaber: ESKA Implants GmbH & Co., 23556 Lübeck (DE)
(72) Erfinder: Grundei, Hans, Dr., 23558 Lübeck (DE)
(74) Vertreter: Fuchs Mehler Weiss & Fritzsche

(56) Entgegenhaltungen:
- EP-A- 0 211 169
- EP-A- 0 314 951
- EP-A- 0 341 199
- EP-A- 0 420 795
- EP-A- 0 500 477
- EP-A- 0 619 990
- EP-A- 0 628 294
- EP-A- 1 062 922
- WO-A-00/48534
- WO-A-98/26725
- DE-A- 3 918 970
- DE-A- 4 442 559
- DE-C- 4 133 433
- DE-C- 19 931 670
- FR-A- 2 790 662
- US-A- 5 725 588

## Beschreibung

Die vorliegende Erfindung betrifft ein Set für die Erstellung eines Implantates als Ersatz für eine zerstörte Beckenknochenpfanne.

Derartige Sets sind bekannt. Beispielhaft sei hingewiesen auf ein Set, wie es aus der DE 39 18 970 C ersichtlich ist, welche die Merkmale des Oberbegriffs von Anspruch 1 aufweist.

Dieses Set besteht aus einer in eine Beckenknochenpfanne einpressbare kalottenförmige Gelenkpfanne und aus jeweils mindestens einem ersten und zweiten Fixationselement zur Verspannung der Gelenkpfanne in dem Beckenknochen. Ein erstes Fixationselement ist durch einen Durchgriff in der Gelenkpfanne setzbar und dort verschieblich führbar, wobei es radial nach außen weist. Auf der Aussenfläche der Gelenkpfanne ist eine von der metallischen offenzelligen Struktur der Gelenkpfanne umfaßte Montageleiste mit mindestens zwei Fixationslagern vorgesehen, in welche wahlweise ein oder mehrere zweite Fixationselemente einsetzbar sind.

Es gibt Schädigungen im Beckenbereich, bei denen das erwähnte Set nicht mehr zum Einsatz kommen kann, da die beschädigten Knochenbereiche so groß sind, daß die erwähnten Fixationselemente die Defekte nicht mehr überbrücken können. Das mit dem bekannten Set erstellte Implantat ist in solchen Indikationsfällen nicht mehr primär-fixationsfähig, da die Kontaktflächen zwischen dem Beckenknochen und dem Implantat zu klein sind, als daß ausreichend Knochen in die Oberflächenstruktur der Beckenpfanne einwachsen könnte. Der mangelnden Primärfixation folgt dann praktisch zwingend die mangelnde Sekundärfixation durch fehlendes Einorganisieren ausreichenden Knochenmaterials in der Oberfläche des Implantates.

Vor diesem Hintergrund ist es die Aufgabe der vorliegenden Erfindung, ein Set für die Erstellung eines sekundärfixationsfähigen Implantates als Ersatz für eine zerstörte Beckenknochenpfanne anzugeben, das die Erstellung eines Beckenpfannenimplantates erlaubt, welches selbst bei schwersten Zerstörungen durch Sekundärfixation stabil implantiert werden kann.

Gelöst wird diese Aufgabe durch das Set mit den Merkmalen des Anspruchs 1. Weitere vorteilhafte Ausgestaltungen ergeben sich aus den Unteransprüchen.

Demgemäß ist vorgesehen, daß das erwähnte Set aufweist:
- eine in das Acetabulum setzbare Gelenkpfanne mit wenigstens abschnittsweise eine dreidimensionale offenmaschige Raumnetzstruktur tragende Außenseite, die mindestens drei ihre Wandung durchsetzende Bohrungen aufweist, welche auf der Außenseite der Gelenkpfanne durch jeweils einen sphärischen Stutzen eingefaßt sind, und die mit einem in Abweichung von einer idealisierten kalottenförmigen Pfanne nach einer Seite hin ausladenden Bereich ausgebildet ist, derart, daß sie in Aufsicht gesehen eine im wesentlichen eiförmige und in Seitenansicht gesehen eine halbeiförmige Kontur aufweist,
- wenigstens ein langgestrecktes Fixationselement, welches an die Gelenkpfanne im Bereich der mindestens drei Bohrungen koppelbar und so ausgebildet ist, daß das der Außenseite der Gelenkpfanne zuzuwendende Ende konkav ausgeformt ist, derart, daß es mit dem die wenigstens drei Bohrungen einfassenden sphärischen Stutzen ein die Verschwenkung des Fixationselementes ermöglichendes Kugelgelenk ausbildet,
- wenigstens einen Gewindebolzen, der durch die wenigstens eine Bohrung setzbar und mit dem wenigstens einen Fixationselement verschraubbar ist, derart, daß das Fixationselement gegen eine weitere Verschwenkung arretierbar ist.

Erfindungsgemäß besteht das Grundset aus drei wesentlichen Komponenten. Die erste Komponente ist die eiförmig oder ovallär ausgebildete Gelenkpfanne, deren Ausformung es gestattet, bei einem praktisch völlig zerstörten Acetabulum größere Überbrückungen zu bieten. Bei einer rein kalottenförmigen Beckenpfanne wäre ein angestrebter Formenschluß mit dem Acetabulum praktisch nicht erreichbar. Die Gelenkpfanne trägt an ihrer Außenseite eine dreidimensionale offenmaschige Raumnetzstruktur, in welche in bekannter Weise nach Stimulierung Knochenmaterial einwächst und so die Sekundärfixation bewerkstelligt. Das Einfassen der wenigstens drei die Wandung der Gelenkpfanne durchsetzenden Bohrungen durch jeweils eines sphärischen Stutzen ist in Verbindung zu sehen mit der zweiten wesentlichen Komponente, nämlich mit dem wenigstens einen langgestreckten Fixationselement. Durch die Bildung eines Kugelgelenkes zwischen dem sphärischen Stutzen und dem konkav ausgeformten Ende des Fixationselementes ist es möglich, daß das Fixationselement zur Gelenkpfanne große Bereiche von Verschwenkwinkeln einnehmen kann. Fixiert wird das Fixationselement durch einen Gewindebolzen, der durch die betreffende Bohrung in der Gelenkpfanne gesetzt wird und mit dem Fixationselement verschraubt wird.

Operativ geht die Implantation des Implantates wie folgt von statten:
Das zerstörte Acetabulum wird gegebenenfalls durch Meißeln noch etwas ausgeräumt und die Gelenkpfanne so in die Aushöhlung eingesetzt, daß der weitest mögliche Formensschluß zwischen dem Beckenknochen und der Außenseite der Gelenkpfanne zur Ausbildung kommt.

Sodann werden in geeignete Regionen des Beckenknochens, also beispielsweise in Richtung des Kreuzbeines oder Darmbeines - was immer günstiger für eine Platzierung erscheint - eine Bohrung ausgefräst für den Einsatz des Fixationselementes, und zwar durch das Innere der Gelenkpfanne, die in diesem Falle gewissermaßen als Bohrschablone dient. Sämtliche Ausfräsungen zur Aufnahme des Fixationselementes sind bei dem erfindungsgemäßen Konzept kranial gerichtet, da die Fixation ausdrücklich in erster Linie auf Zugkräfte unter Belastung ausgerichtet ist. Sodann entfernt der Operateur die Gelenkpfanne aus dem Restacetabulum und setzt ein oder mehrere Fixationselemente in eine oder mehrere zuvor ausgeräumte Ausfräsungen. Danach setzt der Operateur die Gelenkpfanne wieder in das Restacetabulum um und drückt dieses solange hinein, bis die Fixationselemente durch die betreffenden Bohrungen in der Wandung der Gelenkpfanne wieder sichtbar werden bzw. über eine Einfädelungshilfe, beispielsweise in Form eines Drahtes, erfühlbar sind. Ist dies der Fall, setzt der Operateur pro Fixationselement einen Gewindebolzen durch die betreffenden Bohrungen und verschraubt den Gewindebolzen mit dem Fixationselement, und zwar so, daß Zugkräfte auf die Gelenkpfanne übertragbar sind.

Die Ausbildung des sphärischen Stutzens an der Außenseite der Gelenkpfanne zusammen mit der konkaven Ausformung des Ankopplungsendes des Fixationselementes gestattet es, daß beide Teile ihre optimale, spannungsfreie Lage zueinander selbstätig finden. Erst die Verschraubung mit dem Gewindebolzen stellt die dauerhafte Verbindung zwischen Gelenkpfanne und Fixationselement her, und zwar so, daß Kräfte und Momente übertragen werden können. Das so in situ erstellte Implantat ist auf die Sekundärfixation angewiesen, daß heißt auf das Einorganisieren von Knochmaterial in die Außenflächen der Implantatteile. Dies bedeutet, daß das so versorgte Becken nicht unmittelbar nach der Operation belastbar ist, sondern erst nach einigen Wochen. Dies stellt aber für die versorgten Patienten immer noch einen erheblichen Gewinn an Lebensqualität gegenüber dem voroperativen Zustand dar.

Wo es angebracht und möglich erscheint, können zusätzliche Fixationselemente vorgesehen sein, die durch die übrigen Bohrungen in der Gelenkpfanne setzbar sind. Diese Fixationselemente können bevorzugt Knochenschrauben sein. Diese dienen jedoch nicht einer etwaigen Primärfixation des Implantates in dem Restacetabulum, sondern vielmehr einer Verspannung der Gelenkpfanne in situ, um nämlich das Knochenwachstum anzuregen und damit die Einorganisierung in den Außenseiten der Implantatteile.

Bevorzugt sind die wenigstens drei Bohrungen so ausgebildet, daß sie sich zum Inneren der Gelenkpfanne hin konisch erweitern. Dann ist der Kopf des Gewindebolzens bevorzugt als Senkkopf ausgebildet. Diese Kombination gestattet ein Hin- und Herschwenken des Gewindebolzens in der Bohrung in der Gelenkpfanne.

Das Fixationselement besteht bevorzugt aus einem dünnwandigen hohlen Armierungskörper mit einer solchen großen Anzahl von Durchbrechungen in seiner Außenwandung, daß das Verhältnis der Gesamtflächen der Durchbrechungen zu seiner Gesamtoberfläche wenigstens 1:2 beträgt. Nicht nur das Einorganisieren von Knochenmaterial in das Innere des Fixationselementes zur Sekundärfixation des Gesamtimplantates spielt hierbei eine Rolle, sondern auch die federnde Ausbildung der Wandung des Fixationselementes, wie mit dem Ausdruck der Dünnwandigkeit zum Ausdruck kommt. Dadurch wird das ganze System flexibler und seine Verträglichkeit wird erhört. Die federnde Auslenkung der Wandung des Fixationselementes bewegen sich im Mikrometerbereich.

Die Erfindung wird anhand eines Ausführungsbeispiels näher erläutert. Hierbei zeigt:
- Figur 1 die Aufsicht auf den Polbereich der Gelenkpfanne,
- Figur 2 die Seitenansicht des Implantates aus Figur 1,
- Figur 3 das mit dem Gewindebolzen verschraubte Fixationselement, und
- Figur 4 eine Schnittansicht des Implantates aus Figur 2.

Nachfolgend sind gleiche Teile mit denselben Bezugszeichen versehen.

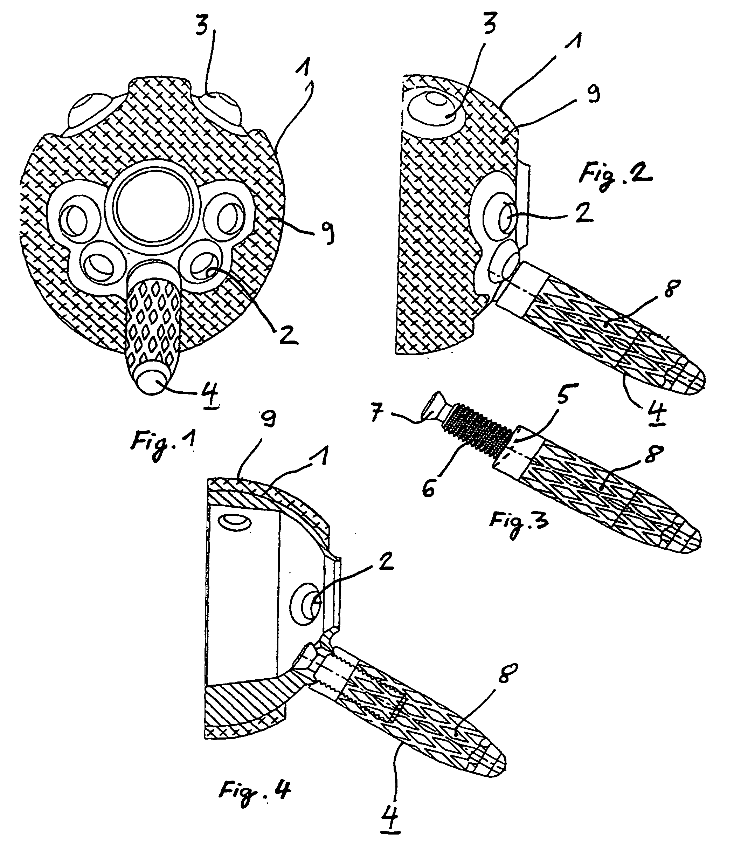

Einen ersten Überblick verschafft Figur 1. Zu erkennen ist die Gelenkpfanne 1 mit ihrer weitgehend mit einer dreidimensionalen offenmaschigen Raumnetzstruktur 9 belegten Außenseite. In Abweichung von einer idealisierten kalottenförmigen Pfanne ist die ovalläre Gelenkpfanne als nach einer Seite hin ausladend ausgebildet, in Figur 1 nach oben hin. Sechs Bohrungen 2 durchsetzen die Wandung der Gelenkpfanne 1. Die Bohrungen 2 sind an der Außenseite der Gelenkpfanne jeweils durch einen sphärischen Stutzen oder Kugelsegment 3 eingefasst. Der sphärische Stutzen 3 ist im Zusammenhang zu sehen mit dem Fixationselement 4. Dieses weist an seinem Ankopplungsende nämlich eine konkave Ausformung 5 (Figur 3) auf, welche zusammen mit dem sphärischen Stutzen 3 nach Art eines Kugelgelenkes wirkt. Dies ermöglicht es dem Fixationselement eine veschwenkte Lage in Bezug auf die Gelenkpfanne 1 einzunehmen, ohne dabei den Formenschluß zwischen Gelenkpfanne 1 und Fixationselement 4 zu verlieren.

Der Kopf 7 des Gewindebolzens 6 ist vorliegend als Senkkopf ausgebildet und arbeitet damit mit den sich zum Inneren der Gelenkpfanne hin verjüngenden Bohrungen 2 zusammen im Sinne einer quasi kardanischen Aufhängung des Fixationselementes 4 an der Gelenkpfanne 1.

Es können mehr als ein Fixationselement 4 an die Gelenkpfanne 1 angekoppelt werden, je nach Krankheitsbild und nach vorhandenen, belastungsfähigen Knochen des Beckens. In die verbleibenden Bohrungen 2 können vor allem in Richtung kaudal Knochenschrauben (nicht dargestellt) eingesetzt werden und mit dem Restknochen verschraubt werden, um so die Gelenkpfanne im Restacetabulum unter Druck zu setzen, was wiederum zur Reizung des umgebenden Knochenmaterials führt, wodurch aber das Einorganisieren von Knochenpartikeln in die Raumnetzstruktur 9 begünstigt, die Einheilphase also verkürzt wird.

## Patentansprüche

1. Set für die Erstellung eines Implantates als Ersatz für eine zerstörte Beckenknochenpfanne, aufweisend
- eine in das Acetabulum setzbare Gelenkpfanne (1) mit wenigstens abschnittsweise eine dreidimensionale offenmaschige Raumnetzstruktur (9) tragende Außenseite, die mindestens drei ihre Wandung durchsetzende Bohrungen (2) aufweist, **dadurch gekennzeichnet, daß** die Bohrungen auf der Außenseite der Gelenkpfanne (1) durch jeweils einen sphärischen Stutzen (3) eingefaßt sind, und die mit einem in Abweichung von einer idealisierten kalottenförmigen Pfanne nach einer Seite hin ausladenden Bereich ausgebildet ist, derart, daß sie in Aufsicht gesehen eine im wesentlichen eiförmige und in Seitenansicht gesehen eine halbeiförmige Kontur aufweist,
- wenigstens ein langgestrecktes Fixationselement (4), welches an die Gelenkpfanne (1) im Bereich der mindestens drei Bohrungen (2) koppelbar und so ausgebildet ist, daß das der Außenseite der Gelenkpfanne (1) zuzuwendende Ende konkav ausgeformt ist, derart, daß es mit dem die wenigstens drei Bohrungen (2) einfassenden sphärischen Stutzen (3) ein die Verschwenkung des Fixationselementes (4) ermöglichendes Kugelgelenk ausbildet,
- wenigstens einen Gewindebolzen (6), der durch die wenigstens eine Bohrung (2) setzbar und mit dem wenigstens einen Fixationselement (4) verschraubbar ist, derart, daß das Fixationselement (4) gegen eine weitere Verschwenkung arretierbar ist.

2. Set nach Anspruch 1, bei dem zusätzliche Fixationselemente vorgesehen sind, die durch die übrigen Bohrungen in der Gelenkpfanne setzbar sind.

3. Set nach Anspruch 1 oder 2, bei dem die wenigstens drei Bohrungen (2) sich zum Inneren der Gelenkpfanne (1) konisch erweitert.

4. Set nach Anspruch 3, bei dem der Kopf (7) des Gewindebolzens (6) als Senkkopf ausgebildet ist.

5. Set nach einem der Ansprüche 1 bis 4, bei dem das Fixationselement (4) aus einem dünnwandigen hohlen Armierungskörper mit einer solchen großen Anzahl von Durchbrechungen (8) in seiner Außenwandung besteht, daß das Verhältnis der Gesamtflächen der Durchbrechungen zu seiner Gesamtoberfläche wenigstens 1:2 beträgt.

## Claims

1. Set for constructing an implant for replacing a damaged acetabular cavity, comprising
- a socket (1) insertable into the acetabulum and with an exterior which, at least in some areas, supports a three-dimensional open-mesh network structure (9) and which has at least three holes (2) extending through its wall,
**characterized in that**, on the exterior of the socket (1), the holes are bordered by in each case a spherical support (3), and which socket is designed with an area protruding laterally outwards in deviation from an idealized cap-shaped socket in such a way that, when viewed from above, it has a substantially ovoid contour and, when viewed from the side, a semi-ovoid contour,
- at least one elongate fixation element (4) which can be coupled to the socket (1) in the area of the at least three holes (2) and is designed such that the end to be directed towards the exterior of the socket (1) is of concave configuration, so that, together with the spherical support (3) bordering the at least three holes (2), it forms a ball joint which permits the pivoting of the fixation element (4),
- at least one threaded bolt (6) which can be fitted through the at least one hole (2) and can be screwed to the at least one fixation element (4) in such a way that the fixation element (4) can be locked against further pivoting.

2. Set according to Claim 1, in which additional fixation elements are provided which can be fitted through the other holes in the socket.

3. Set according to Claim 1 or 2, in which the at least three holes (2) widen conically towards the interior of the socket (1).

4. Set according to Claim 3, in which the head (7) of the threaded bolt (6) is designed as a countersunk head.

5. Set according to one of Claims 1 to 4, in which the fixation element (4) is composed of a thin-walled hollow reinforcement body having such a large number of apertures (8) in its outside wall that the ratio of the total areas of the apertures to its total surface area is at least 1:2.

## Revendications

1. Ensemble pour réaliser un implant de remplacement d'une cavité acétabulaire endommagée, présentant :
- une cavité articulaire (1) pouvant être placée dans l'acétabule, comprenant un côté extérieur portant au moins en partie une structure en réseau spatial (9) à mailles ouvertes tridimensionnelle, qui présente au moins trois alésages (2) traversant sa paroi, **caractérisé en ce que** les alésages sont creusés du côté extérieur de la cavité articulaire (1) à travers à chaque fois un embout sphérique (3) et la cavité articulaire étant réalisée avec une région partant en porte à faux vers un côté, en s'écartant d'une cavité en forme de calotte idéal, de telle sorte qu'elle présente en vue de dessus un contour de forme essentiellement ovoïdale et en vue de côté un contour de forme semi-ovoïdale,
- au moins un élément de fixation longitudinal (4), qui peut être accouplé à la cavité articulaire (1) dans la région des au moins trois alésages (2) et qui est réalisé de telle sorte que l'extrémité à tourner vers le côté extérieur de la cavité articulaire (1) ait une forme concave, de telle sorte qu'elle constitue avec l'embout sphérique (3) incorporant les au moins trois alésages (2) une articulation à rotule permettant le pivotement de l'élément de fixation (4),
- au moins un boulon fileté (6), qui peut être enfoncé à travers l'au moins un alésage (2) et qui peut être vissé avec l'au moins un élément de fixation (4), de telle sorte que l'élément de fixation (4) puisse être bloqué contre un pivotement supplémentaire.

2. Ensemble selon la revendication 1, dans lequel des éléments de fixation supplémentaires sont prévus, lesquels peuvent être enfoncés à travers les autres alésages dans la cavité articulaire.

3. Ensemble selon la revendication 1 ou 2, dans lequel les au moins trois alésages (2) s'élargissent coniquement vers l'intérieur de la cavité articulaire (1).

4. Ensemble selon la revendication 3, dans lequel la tête (7) du boulon fileté (6) est réalisé en tant que tête conique.

5. Ensemble selon l'une quelconque des revendications 1 à 4, dans lequel l'élément de fixation (4) se compose d'un corps de renforcement creux à parois minces avec un nombre d'ouvertures (8) dans sa paroi extérieure telle que le rapport de la surface totale des ouvertures à la surface totale du corps vaille au moins 1:2.
